# EUROPEAN PATENT SPECIFICATION

(11) **EP 2 857 019 B1**
(45) Date of publication and mention of the grant of the patent: **27.05.2020**
(21) Application number: 14194213.6
(22) Date of filing: 29.04.2013
(51) Int. Cl.: A61K 31/4188, A61P 25/28

(54) **Method of treating multiple sclerosis**
Verfahren zur Behandlung von Multipler Sklerose
Procédé de traitement de la sclérose en plaques

(30) Priority: 26.07.2012 FR 1257254; 04.10.2012 US 201213644615
(43) Date of publication of application: 08.04.2015
(62) Divisional of application: 13719836.2
(73) Proprietor: ASSISTANCE PUBLIQUE, HOPITAUX DE PARIS, 75004 Paris (FR)
(72) Inventor: Sedel, Frédéric, 75020 PARIS (FR)
(74) Representative: Flesselles, Bruno F.G.

(56) References cited:
- WO-A1-2011/124571
- BG-B1- 66 011
- DARIN ET AL: "3-Methylcrotonyl-CoA Carboxylase Deficiency and Severe Multiple Sclerosis", PEDIATRIC NEUROLOGY, ELSEVIER SCIENCE, NL, vol. 36, no. 2, 31 January 2007 (2007-01-31), pages 132-134, XP005867283, ISSN: 0887-8994, DOI: 10.1016/J.PEDIATRNEUROL.2006.09.007
- YANG YANLING ET AL: "Spinal cord demyelination associated with biotinidase deficiency in 3 Chinese patients", JOURNAL OF CHILD NEUROLOGY, DECKER PERIODICALS, HAMILTON, CA, vol. 22, no. 2, 1 February 2007 (2007-02-01), pages 156-160, XP008126453, ISSN: 0883-0738, DOI: 10.1177/0883073807300307
- MOHAMMAD SHIRAZI M ET AL: "Dietary supplementation in Iranian multiple sclerosis patients", JOURNAL OF MEDICAL SCIENCES 20070401 PK, vol. 7, no. 3, 1 April 2007 (2007-04-01), pages 413-417, XP002689602, ISSN: 1682-4474
- ANAGNOSTOULI M ET AL: "1-31-03 Biotin in CSF and serum in patients with multiple sclerosis", JOURNAL OF NEUROLOGICAL SCIENCES, ELSEVIER SCIENTIFIC PUBLISHING CO, AMSTERDAM, NL, vol. 150, 1 September 1997 (1997-09-01), page S47, XP027386176, ISSN: 0022-510X [retrieved on 1997-09-01]
- Cathy Breedon: "Nutrition Issues in Multiple Sclerosis", Aunt Cathy's Guide to Nutrition, 1 January 2009 (2009-01-01), pages 1-29, XP055048445, Retrieved from the Internet: URL:http://talwd.org/pdf/fwtexastour/Aunt C MS Handout pt version W REFS 09this.pdf [retrieved on 2012-12-20]

## Description

The invention relates to the treatment of multiple sclerosis, and in particular of the progressive forms and of the neurological sequelae after attacks of the disease, wherein said treatment with biotin has a duration of at least 3 months.

Multiple sclerosis (MS) is a frequent and disabling neurological disease characterized by multifocal destruction of central nervous system myelin.

The prevalence of MS in Europe is approximately 1/2000 inhabitants (Noseworthy et al., 2000). The disease typically begins between the ages of 20 and 30 and affects twice as many women as men. In 80% of cases, the disease initially evolves through attacks which result completely or with sequelae in a few weeks or months (pure remitting phase or emitting phase with sequelae). However, in 40% to 70% of cases, patients who experience an initially remitting evolvement subsequently evolve towards a progressive form (secondary progressive form). In 20% of patients, the evolvement is immediately progressive without attacks (primary progressive form).

For patients who experience an evolvement via regressive attacks, the remissions are less complete over time, resulting in functional sequelae, the ability to walk being lost on average 20 years after the beginning of the disease.

Thus, the conventional form of multiple sclerosis can have three evolutive modes:
- Relapsing-remitting form: exacerbations alternating with remissions during which partial or total recovery is observed. The remissions can last months or years. The exacerbations can occur spontaneously or be triggered by certain external factors, such as an infection, post-partum or certain vaccinations.
- Primary progressive form: The disease evolves progressively without remissions, with the possibility of evolutive plateau during which the disease does not progress. Contrary to the cyclic tendency, there are no clear exacerbations.
- Secondary progressive form: This form follows on from a remitting form which begins with attacks alternating with remissions, followed by a gradual progression of the disease without identifiable attacks.

Pyramidal syndrome marks the beginning of (reveals) the disease in 20% of cases, and manifests itself through walking problems with high fatigability, spasticity, exaggerated reflexes in the lower limbs. At the end of the attack, the Babinski sign often remains as a sequela.

Retrobulbar optic neuritis is also an indication of the disease in close to a third of cases: it is the most evocative symptom. It manifests itself for the patient through a rapid and profound decrease in visual acuity, ocular and orbital pain, increased with eye movements, central or cecocentral scotoma and colour blindness (dyschromatopsa of the red-green axis). At the acute stage, the back of the eye is normal, and it is only after about 15 days that atrophy of the papilla occurs, testifying to the damage to the optic nerve and sometimes persistent as a sequela. The visual evoked potentials are impaired, with slowing of the P100 wave.

Sensory problems are common. They are essentially subjective: paresthesia, pins and needles, Lhermitte's sign (electric shock sensation running down the spine when flexing the neck). A posterior cordonal syndrome with deep sensory disorders is sometimes found, and more rarely involvement of the spinothalamic tract with thermalgesic anaesthesia. Facial pain (or, conversely, anaesthesia) is possible in the event of the trigeminal nerve being affected in its bulbar portion.

The disease may also manifest itself through:
- A vestibular syndrome combining rotary vertigo, nystagmus and ataxia;
- A cerebellar syndrome. Demyelinated plaques are frequent in the cerebellum and in the posterior fossa, which can produce a cerebellar syndrome with an unstable upright stance, walking as if inebriated, movements which are uncoordinated, etc.;
- Diplopia consisting of a sensation of double vision due to paralysis of one or more oculomotor muscles. Internuclear ophthalmoplegia is possible in the event of involvement of the posterior longitudinal bundles, which manifests itself in the lateral gaze through an incomplete adduction of one eye associated with nystagmus of the eye in abduction;

- Genito-sphincteric disorders are frequent and are linked to spinal cord involvement. They manifest themselves through urinary urgency (or urinary retention), constipation and impotence. These disorders are a source of acute urine retention, and urinary infections;
- Facial paralysis;
- Asthenia (fatigue), a frequent symptom of multiple sclerosis, is sometimes the one which is the most debilitating.

Multiple sclerosis is generally considered to be an autoimmune disease which occurs on a particular genetic background (Weiner, 2004; Chaudhuri et al., 2004). From the neuropathological point of view, the disease is characterized by demyelinated plaques, well-defined hypocellular regions, within which are observed a scarcity of myelin, an astrocytic gliosis and sometimes an inflammatory infiltrate which, when it is present, attests to the active nature of the disease. With time (but sometimes early on), there are also irreversible axonal lesions, the mechanism of which is poorly understood.

Thus, it is possible to distinguish two components in the physiopathology of multiple sclerosis: (1) an inflammatory component, responsible for the evolutive attacks, and beginning with the arrival of CD4+ T lymphocytes in the central nervous system (Weiner, 2004), and (2) a degenerative component, the mechanism of which is for the moment poorly understood (Chaudhuri et al., 2004) and characterized by progression with few inflammation.
Recently, it has been hypothesized that this progressive neurodegeneration might be associated with secondary energy failure. Indeed, it is believed that, in the normal condition, myelin insulation reduces the energy demand during impulse propagation because ATP is needed to reform the resting membrane potential only at the Ranvier nodes. Demyelinated fibres are placed at an energetic disadvantage because of increased ionic leaks across the denuded axon membrane, resulting in an increased energy demand for ion pumping. In addition, energy production may be compromised owing to mitochondrial disruption and Na⁺-K⁺-ATPase-mediated ion transport may be reduced in many demyelinated axons in the MS brain, which could bias such an axon towards a state of 'virtual hypoxia'. The resulting mismatch between energy supply and demand could culminate in degeneration (Stys et al. 2012).

While immunosupressive and immunomodulatory drugs that target the inflammatory component of the disease have been shown to decrease the number of relapses and of brain lesions, there is still a debate whether these drugs are truly efficient in preventing the long term handicap's progression. Nowadays, there is no efficient drug acting on the progressive phase (whatever primary or secondary) of the disease.

Interferon beta and glatiramer acetate have proven to be effective in multiple sclerosis (attacks less numerous and less severe, improvement of lesions visible by MRI, sometimes a less evolutive nature of the handicap).

The indications for interferon treatment are remitting MS with at least two attacks over the previous two or three years, or secondary progressive MS with persistence of attacks (continuous and progressive worsening, without remission between the acute phases). The current tendency is to begin the treatment early, as soon as the first attack occurs under certain conditions since it could then reduce the functional sequelae. However, the long-term efficacy remains disputed (Filippini G, Munari L, Incorvaia B et al. Interferons in relapsing remitting multiple sclerosis: a systematic review [archive], Lancet, 2003; 361: 545-552).

Glatiramer acetate, for its part, is a copolymer consisting of several amino acids. It appears to space out the attacks in ambulatory patients (who can still walk on their own) suffering from multiple sclerosis evolving through attacks, of relapsing/remitting type, characterized by at least two attacks over the course of the previous two years, as effectively as interferon. It appears to act by causing immune tolerance of the lymphocytes with respect to myelin constituents.

Natalizumab (Tysabri®) is a monoclonal antibody directed against the leukocytes integrin alpha-chain. It can be proposed in remitting MS, either as frontline treatment in severe cases (two attacks in one year with sequelae) or after failure of interferons (one attack in one year despite the treatment).

Gilenya® (fingolimod) belongs to the sphingosine-1-phosphate (S1P) receptor modulator class. The indications are the same as those of natalizumab, namely the remitting forms with attacks after failure of interferons. An international trial is currently ongoing in order to evaluate the efficacy in the primary progressive forms of MS (results not available).

Fampyra® is a preparation of fampridine (4-aminopyridine, 4-AP or dalfampridine) in the form of a sustained-released tablet. This medicament is indicated for the treatment of the sequelae, in particular of the problems in walking which occur during the remitting forms with sequelae or the progressive forms of the disease. Studies have shown that Fampyra® improves walking in a small proportion of patients. Fampyra® is considered as a symptomatic drug in a subset of patients and not as a disease modifying therapy.

In the severe forms, the use of immunosuppressants, among which mitoxantrone, which is more effective than corticoids, but which comprises many more side effects, can be proposed.

Social and psychological care is necessary, through integration into patient groups, keeping a job and, as required, adaptation of the workstation, psychotherapy, treatment for depression or for an anxious state.

It is important to underline that, while the immunosuppressant or immunomodulatory treatments which are aimed at inhibiting the inflammatory reaction are effective, at the beginning of the disease, in decreasing the number or the duration of the active lesions, they have only very little effectiveness on the long-term handicap and have only little or no effectiveness in the progressive (primary or secondary) forms of the disease. As regards the sequelae, only Fampyra® looks to be a medicament capable of improving walking in certain patients.

Biotin (or vitamin H) is a ubiquitous water-soluble vitamin which is naturally found in many foods, such as offal, eggs and certain vegetables. In mammals, biotin acts as a cofactor for four metabolism carboxylases involved in several key steps of energy metabolism, including pyruvate carboxylase (neoglucogenesis), 3-methylcrotonyl CoA and propionyl CoA carboxylases (catabolism of certain amino acids which supply the Krebs cycle with intermediate metabolites), and acetyl CoA carboxylase (fatty acid synthesis).
Over the past few years, it has also been shown that biotin can regulate the expression of numerous genes via a mechanism of biotinylation/debiotinylation of histones, which are protein structures that regulate DNA conformation and, in so doing, the access of certain regions of the genome to transcription factors. It appears that a large number of genes of which the expression is regulated by biotin encode proteins involved in energy metabolism (Zempleni et al., 2009). Consequently, the mechanism of action of biotin can bee seen as an enhancer of brain energy (ATP) production.
In view of the results herein reported showing that biotin has positive effects in progressive multiple sclerosis with improvement of neurological disability, even after a long period of motor or visual deficit, this compound is thus a good candidate to enhance functional recovery in AMN and following ischemic stroke. In the case of ischaemic stroke, It is expected that this effect may occur not only in acute/subacute phases of the ischaemic attack but also in neurological sequelae observed later on.

Patent application WO 2011/124571 describes the use of biotin at a high dose (of the order of 100 to 600 mg/day) for the treatment of visual impairments, in particular related to optic atrophy. It should be noted that the visual impairments actually described in this application are symptoms related to a particular leukoencephalopathy, i.e. an involvement of the white matter of the brain. This document neither describes nor suggests that biotin could be used for the treatment of multiple sclerosis. Indeed, even though certain symptoms may be similar (visual problems), the etiology is different.

Although biotin is indicated in children with a deficiency in biotinidase, methylcrotonyl CoA carboxylase or in holocarboxylase synthase, pathology conditions which are sometimes associated with leukoencephalopathy or with optic neuropathy, the doses necessary are of the order of 10 mg/day during these diseases (review in Wolf, 2010).

Darin et al. have reported a child with multiple sclerosis and a 3-methylcrotonyl-CoA carboxylase deficiency.
This patient had a severe inflammatory form of multiple sclerosis but not the so-called "primary or secondary" multiple sclerosis which is a less inflammatory and more progressive degenerative disease. The child had been treated with different compounds including biotin. However, in this case report, biotin had been administered to treat the associated condition i.e. 3-methylcrotonyl-CoA carboxylase deficiency, which is well known to respond to biotin in some instances (Baumgartner M) but there was no intention to treat multiple sclerosis with biotin. In the case report, the products that were administered to the patient for treating the multiple sclerosis are classical immunosuppressive drugs including mitoxantrone and steroids.

In the context of the present invention, it has, in fact, been shown that biotin, in particular at a high dose, can make it possible to improve the condition of patients suffering from multiple sclerosis, particularly progressive forms of multiple sclerosis or of neurological sequelae after attacks of the disease. In contrast, it is possible that biotin has no effect on inflammatory relapses since at least two patients exhibited relapses while on treatment (patients n°2 and 3).

As it will be seen in the examples, although this improvement has been observed in four patients with chronic optic neuropathy related to MS, biotin can be used in patients exhibiting other syndromes, the etiology of the disease remaining the same (demyelinization). This is confirmed by the results obtained in a patient with homonymous lateral hemianopsia caused by damage to the cerebral optic radiations and in one patient with progressive tetraplegia caused by a lesion of the upper cervical spinal cord.

The invention thus relates to biotin for use thereof in the treatment of progressive multiple sclerosis especially in the primary and secondary progressive forms of the disease and in the treatment or neurological sequelae after attacks of the disease, wherein said treatment with biotin has a duration of at least 3 months.

Also described are compositions containing biotin for the use thereof in the treatment of multiple sclerosis, and also the use of biotin for the production of a drug intended for the treatment of multiple sclerosis. The teachings of the disclosure thus make it possible to implement treatment methods comprising the administration of biotin to patients suffering from multiple sclerosis.

The biotin is used for the treatment of the progressive forms of multiple sclerosis (primary or secondary progressive forms), wherein said treatment with biotin has a duration of at least 3 months.

Likewise, is herein disclosed the use of the biotin, in the treatment of multiple sclerosis, in order to allow treatment of the sequelae observed in the relaxing/remitting form, after the attacks.

It can be used alone or in combination with another compound used for treating multiple sclerosis, in particular a compound as described above. The disclosure therefore exemplifies a composition containing biotin and also another medicament against multiple sclerosis, for simultaneous, separate or sequential (spread out over time) use in the treatment of multiple sclerosis.

The biotin is preferentially administered at a high dose, i.e. at a dose greater than 50 mg per day. Even if a maximum dose is not really envisaged, the latter should not generally exceed 500 mg, 600 mg or 700 mg per day. In that way, a dose at least equal to 1 mg/kg/day, preferably 3 mg/kg/day, preferably 5 mg/kg/day, or at least equal to 7.5 mg/kg/day, or even around 10 mg/kg/day, is administered to the patient. Between 50 and 700 mg of biotin per day are thus administered to the patients, generally between 50 and 500 mg per day, or between 50 and 600 mg per day, more preferably between 100 and 300 mg per day, generally around 300 mg per day. One can thus administered at least 50 mg par day, more preferably at least 100 mg per day, or at least 150 mg per day, or even 200 or 250 mg per day.

In one particular embodiment which is preferred (in particular for problems of ease of use by the patient), the biotin is in a form suitable for oral administration. This therefore involves a composition for oral administration, which will contain at least 20 mg, preferably at least 40 mg of biotin, or even 50 mg, 75 mg, 100 mg, 150 mg or 250 mg of biotin. This composition is preferentially for pharmaceutical use, and is therefore a medicine. It is understood that each unit dose of this composition contains at least 20 mg, preferably at least 40 mg, or even 50 mg, 100 mg, 150 mg or 250 mg of biotin, as active ingredient.

In one particular embodiment, this composition for oral administration contains biotin as sole active ingredient, and also excipients, without any other active ingredient.

An excipient should be understood to mean any compound forming part of the formulation which is intended to act as a simple support, i.e. which is not intended to have a biological activity.

This composition can be in any form known in the art. In particular, it is in the form of gel capsules, tablets (optionally film-coated), pills or lozenges. In another embodiment, it is in the form of a syrup. Said syrup contains an amount such that it contains at least 20 mg, preferably at least 40 mg, or even 50 mg, 75 mg or 100 mg of biotin per unit dose. The concentration of biotin in this syrup depends on the unit dose that it is desired to give to the patient.

Excipients which can be used by those skilled in the art are well known in the art. Talc (E553b), microcrystalline cellulose, lactose, mannose, starch (in particular corn starch), magnesium stearate (E572) and stearic acid (E570) can thus be chosen. This list is not exhaustive.

When this composition is prepared in the form of 25 gel capsules, a preferred excipient is microcrystalline cellulose.

When the composition is in the form of a film-coated tablet, said film-coating may be formed from any substance known in the art, such as hypromellose (E464), ethylcellulose, macrogol, talc (E553b) titanium dioxide (E171) or iron oxide (E172).

The active ingredient may also be colored (by any acceptable coloring, such as cochineal), thereby making it possible to verify that the biotin is well dispersed in the excipient.

A slow release (or slow sustained) form may also be envisaged given the fact that plasma half life of biotin is short (about 2 hours).
Said slow release compositions are known in the art and described in particular in WO 2011/077239. In particular, said slow release compositions may comprise a slow release matrix comprising biotin alone or with one or more active ingredient(s).
In a specific embodiment, the slow release composition comprises a matrix allowing immediate release, wherein said matrix comprises biotin alone or with one or more other active ingredient(s) and the slow release is achieved by a release modifying matrix or coating.
Thus, the slow release composition may provide immediate release and differed (slow) release of biotin.
In a specific embodiment slow release may be achieved through an osmotically driven release system.
In another embodiment, the slow release composition comprises a core comprising biotin, optionally one or more active ingredient(s), and optionally pharmaceutical excipient(s) and one or more outer layers, wherein the outer layers comprises one or more slow release agent(s).

In another aspect, the biotin may be in the form which allows administration by injection: this then involves an injectable composition containing at least 20 mg, preferably at least 40 mg, or even 50 mg, 75 mg, 100 mg, 150 mg or 250 mg of biotin per unit dose.

This injectable composition may be in the form of a vial containing the biotin, and also acceptable excipients. The concentration of biotin is adjusted according to the envisaged volume of the vial. Certain excipients which improve biotin solubility can be used.

The excipients that can be used for the production of injectable compositions are well known in the art. Mention may in particular be made of sodium dihydrogen phosphate, sodium bicarbonate (E550i), methyl para-hydroxybenzoate (E218) and propyl para-hydroxybenzoate (E216), which can be used together in proportions that those skilled in the art are capable of determining. The water used is water for injection. The injection is preferably carried out intramuscularly. It can also be carried out intravenously.

### Description of the figure

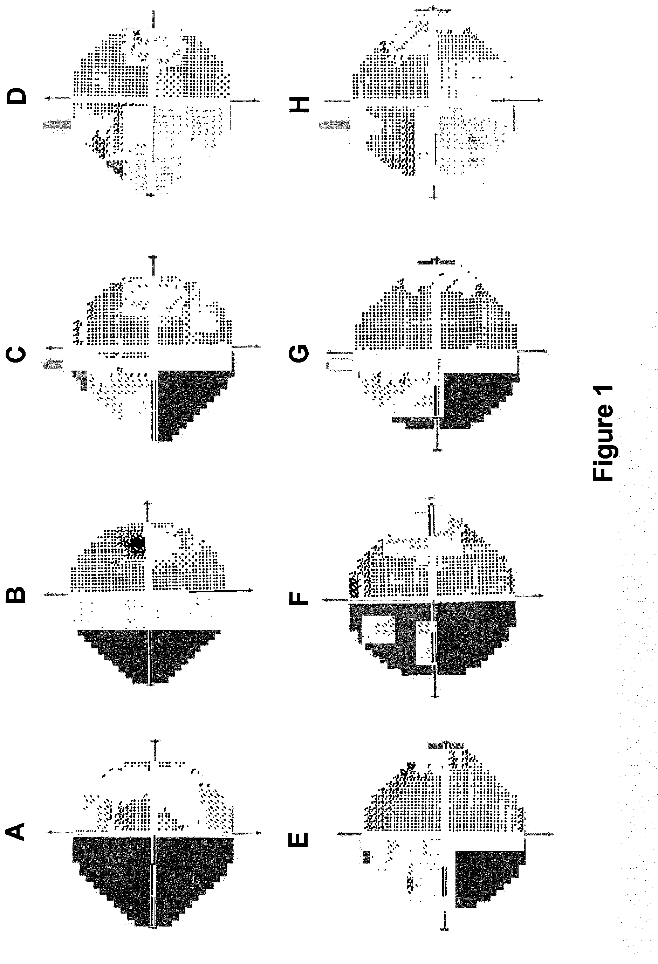

Figure 1: Change in the visual fields (automated campimetry of Humphrey) of patient 5 before and after treatment with biotin (100 mg/day) begun on April 12, 2012. A to D: right eye; E to H: left eye. A, E: November 2010; B, F: January 2012; C, G: 30 June 2012; D, H: April 2013. The absence of spontaneous modification of the visual field between November 2010 (A, E) and January 2012 (B, F), i.e. before treatment initiation, should be noted. The improvement in the visual field in the upper left quadrant in June 2012, two months after introduction of the biotin treatment, should be noted (C and G). The improvement is marked by lightening of the computer plot (arrows). The improvement is much more pronounced after 1 year of treatment (D and H) with an almost complete recovery in the right and left upper visual field quadrants (arrows).

### Examples

Six patients with primary or secondary progressive forms of multiple sclerosis received biotin. All of them improved over a treatment period of 3 to 12 months.

### Description of clinical cases

### Patient 1

This 72-year-old patient had progressive multiple sclerosis with optic involvement: right predominant rapid visual acuity decrease approximately three years before the treatment.

Three months after the beginning of these problems, the patient received 3 infusions of corticoids which led to a significant but transient improvement in her visual acuity. Two further series of infusions were carried out 5 and 7 months after the beginning of the sight problems, without any notable effect.

Nine months after the beginning of the problems, the visual acuity continued to decrease, going to 1/10 on the right and 5/10 on the left. Eleven months after the beginning of the problems, she could only count fingers on the right and the visual acuity went to 2/10 on the left.

The patient also had paroxysmal problems with walking, described as balance problems associated with lower limb weakness which each time lasted less than 24 hours.

Two years after the beginning of the problems, treatment with biotin was begun at the dose of 100 mg three times a day.

Three months later, the patient noted an improvement in her visual acuity: she could read telephone numbers, she could make out faces and could read newspaper headlines. The visual acuity was noted at 2/10 on the left and 5/10 on the right. Balance was more assured, in particular when turning round. She could cook alone, which was not the case previously. The MRI was unchanged, as was the brain MRI spectro. On the other hand, the visual evoked potentials showed the reappearance of a P100 wave on the left (no response was noted on the right) of prolonged latency (126.5 ms).

The treatment was continued at the same dose. After six months of treatment, the evoked potentials showed the beginnings of a P100 wave on the right and also an improvement in the latency of the left P100 wave (which went from 126.5 to 111.8 ms). The brain MRI spectro showed a clear decrease in the choline peak and in the choline/creatine ratio. The treatment with biotin was increased to 600 mg/day. After nine months of treatment, a bilateral P100 wave was noted.

The treatment was then continued at the dose of 300 mg/day for 15 months, then 100 mg/day for 9 months.

Between the beginning of the treatment and the date of the last visit, the visual acuity remained stable (2/10 on the left and 5/10 on the right) without any further episode of optic neuropathy. The balance also remained stable.

### Patient 2

A man born in 1987, without any particular personal history. His family history showed that his mother had multiple sclerosis. The patient had a first neurological episode in May 2006, characterized by nystagmus, limb pain and a problem with balance, having regressed in 8 days. A second episode occurred in February 2008, characterized by fatigue, an episodic sensation of double vision and problems with balance, related to a static cerebellar syndrome. The search for an autoimmune disease was negative. The medullary MRI showed an area of hypersignal at the cervical level. The brain MRI showed numerous areas of hypersignals in the periventricular white matter, without taking contrast, the appearance of which is compatible with the diagnosis of multiple sclerosis. Treatment with interferon lb (betaferon) began in February 2008. The patient experienced a further diplopia-type attack in July 2008 which was regressive after Solumedrol® (Methylprednisolone) infusions.

Between 2009 and 2012, without the patient having a further attack of his disease, he indicated a very insidious and progressive decrease in his visual acuity. Although the visual acuity was considered to be normal in 2008, in July 2010 the visual acuity of the right eye was noted at 2/10 and that of the left eye at 6/10. The evoked potentials (July 2010) show a bilateral slowing of the P100 waves to 140 milliseconds, attesting to bilateral involvement of the optic nerves. Three Solumedrol® infusions were carried out in September 2010 and then 4 infusions between 8 and 11 August 2011, without any effect. The visual acuity in December 2011 went to 1/10 on the right and 3/10 on the left. The papillae are bilaterally pale. OCT (Optical Coherence Tomography) shows a considerable fall in the thickness of the peripapillary nerve fibres to 65 microns on the right and 61 microns on the left, confirming the bilateral involvement of the optic nerves. The Goldmann visual field shows two central and cecocentral scotomas and also an enlargement of the blind spot on the right side with a slight degradation compared with the previous visual field. On the left side, the existence of a central scotoma, which is also slightly enlarged compared with the previous visual field, is noted.

The successive MRIs between 2008 and 2011 do not show any increase in the damage load, indicating that the patient has a progressive form of multiple sclerosis characterized by involvement of the optic nerves.

Treatment with biotin was then introduced on 6 March 2012 at the dose of 100 mg/day until 30/03 and then 200 mg/day from 6/04 to 6/05/2012 then 300 mg/day from 6/05/2012 to 7/06/2012. Three complete ophthalmological examinations were carried out during this period of time: a first on 30/03/2012, which showed no improvement in the visual acuity compared with the examination on 6/03 (before treatment), a second on 7/06/2012 (after 3 months of treatment), which showed a very significant improvement in the visual acuity of the left eye, which went from 3/10 to 7.5/10. At third examination in December 2012 (after 9 months of treatment), VA had risen to 3/10 right and 6/10 left.

In November 2012, while on treatment with biotin, the patient exhibited a relapse of multiple sclerosis with diplopia. He recovered after three infusions of steroids (methylprednisolone 1 gr/day).

### Patient 3

Man born in 1980; since 2003, he experienced progressive cerebellar and pyramidal syndrome, combined with a general feeling of fatigue and cognitive impairment with attention difficulties. He had difficulties to see colors on the red-green axis. Progressive evolution with superimposed relapses occurred between 2003 and 2012. Brain MRI showed a periventricular leukoencephalopathy with involvement of the corpus callosum and juxta-cortical white matter. On T2 sequences, there were multiple small nodular high signals, some of which perpendicular to the anteroposterior axis suggesting multiple sclerosis. Lumbar puncture showed inflammatory fluid with 6 éléments/mm³ but without intrathecal synthesis of immunoglobulins. A diagnosis of primary progressive multiple sclerosis was made.

The patient was treated by monthly pulses of IV methylprednisolone (1 gr/month) between April 2008 and April 2009 without a clear benefit. He received interferon β1b (betaferon) between June 2010 and Nov 2010 because of superimposed relapses. However, his maximal walking distance decreased progressively from 2 km in 2008 to 100-200 meters. Progressive loss visual acuity was also noticed with bilateral AV estimated at less than 1/10 in July 2012.

Treatment with biotin was started on 2012/07/12 starting at 100 mg/day for one month, then 100 mg twice a day for one month and then 100 mg three times a day from 2012/09/12. At the beginning of October 2012, the patient noticed that he was able to distinguish colors again, his attention and concentration abilities were better (confirmed by the family) as well as walking speed. On 2013/02/07, after 7 months of treatment, ophthalmological examination confirmed an increased VA from 0.7/10 (before treatment) to 1.2/10 on the right and from 0.6/10 (before treatment) to 1/10 on the left. Visual field analysis using the Goldman method showed a clear improvement on the right already noticed at 4 months of treatment (Nov 2012) and confirmed at 7 months (Feb 2013). No adverse effects of the treatment were noted. In September 2012, while on treatment with biotin, he exhibited a relapse of multiple sclerosis with fatigue and worsening of ataxia. He recovered shortly after three infusions of steroids (methylprednisolone 1 gr/day).

### Patient 4

This 44 year old man had a 4 years history of progressive multiple sclerosis involving optic nerves.

Treatment with biotin was started on Oct 2010. At that time, visual acuity was measured at 3/10 on the right and 0.25 on the left. Visual fields showed bilateral ceoco-central scotoma. After three months of escalating doses of biotin (from 100 to 300 mg), visual acuity increased from 3/10 to 4/10 on the right and from 0.25/10 to 3/10 on the left. The patient noticed subjective improvement. He could read easily with both eye opened (he had to close his left eye while reading before treatment). Furthermore, he experienced Uhthoffs phenomenon before treatment that improved after. No adverse effects were noticed. Visual field analysis using the Godman method showed a clear improvement of the left scotoma, and, to a lesser extend, of the right one

### Patent No. 5

A 29-year-old woman with no personal or family history. In mid-October 2004, she presented a left homonymous lateral hemianposia in relation to an inflammatory lesion of the white matter located on the path of the right optic radiations. The examinations had then revealed intrathecal synthesis of immunoglobulins in the CSF and the evolvement had initially been favourable after 3 infusions of Solumedrol®. The symptomology subsequently reappeared. After 3 infusions of Solumedrol® and orthoptic therapy, the evolvement was marked by a functional improvement. In February 2005, the appearance of further visual problems and of a further right parieto-occipital lesion taking contrast was noted. The evolvement was gradually favourable. However, a left inferior quadrantanopia persisted. The visual acuity was at 6/10^{th} on the right and 7/10^{th} on the left. Between 2005 and the end of 2011, fluctuations in the visual acuity were noted, with regressive periods of worsening on corticoids in November 2005, March 2006 and October 2006, with persistence of a left homonymous lateral heminanopsia between attacks. From the end of 2008, the visual acuity has been stable at 6/10 bilaterally. This stability of the homonymous lateral hemianopsia is demonstrated by several examinations of the visual field carried out between the end of 2010 and the beginning of 2012. In the face of the lack of recovery of the visual acuity, it was decided to begin a treatment with biotin at the dose of 100 mg/day. The visual evoked potentials carried out at this time were normal, demonstrating that the visual impairment is not related to an involvement of the optic nerves themselves, but indeed to a homonymous lateral hemianopsia in relation to the lesions of the white matter of the brain affecting the optic radiations. The treatment with biotin was prescribed on 12/04/2012 at the dose of 100 mg/day. The new examination of the visual field on 30/06/2012 (after 1 and a half months of treatment) shows a clear improvement in the homonymous lateral hemianopsia (Figure 1) that was even more pronounced after 5 months, 9 months and 12 months of treatment.

### Patient 6

This now 73 years old woman had a history of secondary progressive MS. Her disease started with a relapsing phase between age 30 and age 40. Then, from 61 years old, she developed progressive limb weakness starting in lower limbs and then progressing to upper limbs from the age of 65. At age 62, she started to use a cane, at 62, she used two canes, at 63 she had to use a wheelchair and became wheelchair-bound at 64. At 66, clinical examination disclosed tetraparesis. At 68, she experience severe difficulties to eat by herself. Several attempts to stop disease progression using monthly pulses of IV methylprednisolone or cyclophosphamide felt to produce any benefit. All treatments were stopped in 2008, at the age of 68.

Brain and spinal cord MRI disclosed a high signal area involving the upper spinal cord and lower medulla oblongata.

Treatment with biotin (100 mg/day) was started in sept 2012. At that time the patient displayed severe tetraparesis quoted at 1/5 right arm, 3/5 left arm, 0/5 right leg, 1/5 left leg. She also had severe swallowing problems, dysarthria and neck weakness. She used a straw to drink a glass of water and was not able to bring a cup of coffee from the table to her lips. After 2 months of treatment the patient noticed some improvement in the left hand that still progressed over the next 2 months. When she was seen in January 2013 (after 4 months), the weakness had markedly improved, leading to a strength of 2/5 in the right upper limb, 4/5 in the left upper limb, 2/5 in the left lower limb. The patient had no dysarthria and swallowing problems almost disappeared. Neck weakness was no longer present. The patient was now able to bring a cup of coffee with her left hand to her lips. After 6 months of treatment (100mg/day), the patient still improved a little bit: she could move both legs and strength in left upper limb was still better. Movement of the right hand was confirmed. The biotin dosage was increased to 100 mg three times a day. After three weeks at 300 mg/day, strength in the right arm still improved with biceps contraction quoted at 3/5.

### Discussion

It was therefore observed that the clinical condition of patient 1 with a secondary progressive form of multiple sclerosis improved and stabilized under treatment with biotin.

This observation was confirmed in five other patients suffering from multiple sclerosis including three patients with progressive optic neuropathy (patient No. 2,3 and 4), one patient with sequelae consisting of involvement of the optic radiations in the white matter of the brain (patient No. 5) and one patient with progressive tetraplegia/swallowing problems caused by a lesion of the upper cervical spinal cord (patient No. 6). Of note, two patients (n°2 and 3) exhibited a relapse while on treatment with biotin. This may indicate that biotin is not efficient in preventing inflammatory relapses.

To date, these six treated patients have shown an improvement authenticated on several parameters: magnetic resonance spectroscopy, visual evoked potentials, visual acuity, visual field (campimetry), and neurological examination.

In the six cases, the improvement occurred within three months following introduction of the treatment with additional improvement during the next 9 months, while the analysis of the retrospective data showed stability and/or progressive worsening of the visual impairment during the 2 years preceding the introduction of the treatment. In addition to the improvement of existing symptoms, patients did not present any new symptoms.

This represents an important advance since no treatment is currently recognized in the progressive (primary or secondary) forms of multiple sclerosis and also on the symptoms related to sequelae of the disease.

### References

Anagnostouli M, Livaniou E, Nyalala JO, Evangelatos G, Zournas C, Ithakissios DS, Papageorgiou C. Cerebrospinal fluid levels of biotin in various neurological disorders. Acta Neurol Scand. 1999 June; 99(6): 387-92.
Baumgartner M. 3-Methylcrotonyl-CoA carboxylase deficiency. Orphanet encyclopedia, February 2005. http://www.orpha.net/data/patho/GB/uk-MCC.pdf Brainin M, Zorowitz RD. Advances in stroke: recovery and rehabilitation. Stroke. 2013 Feb;44(2):311-3
Carmichael ST. Translating the frontiers of brain repair to treatments: starting not to break the rules. Neurobiol Dis. 2010 Feb;37(2):237-42.
Chaudhuri A, Behan PO. Multiple sclerosis is not an autoimmune disease. Arch Neurol. 2004 Oct; 61(10): 1610-2.
Dabbagh O, Brismar J, Gascon GG, Ozand PT. The clinical spectrum of biotin-treatable encephalopathies in Saudi Arabia. Brain Dev. 1994; 16 Suppl:72-80.
Darin et al Pediatr Neurol. 2007 Feb;36(2):132-4
Dávalos A, Secades J. Citicoline preclinical and clinical update 2009-2010. Stroke. 2011 Jan;42(1 Suppl):S36-9
Debs R, Depienne C, Rastetter A, Bellanger A, Degos B, Galanaud D, Keren B, Fisher M. New approaches to neuroprotective drug development. Stroke. 2011 Jan;42(1 Suppl):S24-S27
Floel A, Cohen LG. Recovery of function in humans: cortical stimulation and pharmacological treatments after stroke. Neurobiol Dis. 2010 Feb;37(2):243-51. Gutiérrez-Fernández M, Fuentes B, Rodriguez-Frutos B, Ramos-Cejudo J, Vallejo-Cremades MT, Díez-Tejedor E. Trophic factors and cell therapy to stimulate brain repair after ischaemic stroke. J Cell Mol Med. 2012 Oct;16(10):2280-90 Lyon-Caen O, Brice A, Sedel F. Biotin-Responsive Basal Ganglia Disease (BBGD) in Europeans with novel SLC19A3 mutations. Arch Neurol. 2010 Jan; 67(1): 126-30.
Noseworthy JH, Lucchinetti C, Rodriguez M, Weinshenker BG. Multiple sclerosis. N Engl J Med. 2000 Sep 28; 343(13): 938-52.
Ozand PT, Gascon GG, Al Essa M, Joshi S, Al Jishi E, Bakheet S, Al Watban J, Al-Kawi MZ, Dabbagh O. Biotin-resonpsive basal ganglia disease: a novel entity. Brain. 1998 Jul; 121 (Pt7): 1267-79.
Lo EH, Dalkara T, Moskowitz MA. Mechanisms, challenges and opportunities in stroke. Nat Rev Neurosci. 2003 May;4(5):399-415.
Polman CH, Reingold SC, Edan G, Filippi M, Hartung HP, Kappos L, et al. Diagnostic criteria for multiple sclerosis: 2005 revisions to the "McDonald Criteria". Ann Neurol. 2005 Dec; 58(6): 840-6.
Ramaekers VT, Brab M, Rau G, Heimann G. (1993) Recovery from neurological deficits following biotin treatment in a biotinidase Km variant. Neuropediatrics 24: 98-102.
Ramos-Cabrer P, Campos F, Sobrino T, Castillo J. Targeting the ischemic penumbra. Stroke. 2011 Jan;42(1 Suppl):S7-11.
Sotgiu S, Pugliatti M, Fois ML, Arru G, Sanna A, Sotgiu MA, Rosasti G. Genes, environment, and susceptibility to multiple sclerosis. Neurobiol Dis. 2004 Nov;17(2):131-43
Stys PK, Zamponi GW, van Minnen J, Geurts JJ. Will the real multiple sclerosis please stand up? Nat Rev Neurosci. 2012;13:507-14.
Subramanian VS, Marchant JS, Said HM. Biotin-responsive basal ganglia disease-linked mutations inhibit thiamine transport via hTHTR2: biotin is not a substrate for hTHTR2. Am J Physiol. 2006; 291(5): 851-859
Vlasova TI, Stratton SL, Wells AM, Mock NI, Mock DM. Biotin deficiency reduces expression of SLC19A3, a potential biotin transporter, in leukocytes from human blood. J Nutr. 2005; 135(1): 42-47.
Weiner HL. Multiple sclerosis is an inflammatory T-cell-mediated autoimmune disease. Arch Neurol. 2004 Oct; 61(10): 1613-5.
Wolf B. Clinical issues and frequent questions about biotinidase deficiency. Mol Genet Metab. 2010 May; 100(1): 6-13
Zempleni J, Wijeratne SS, Hassan YI. Biotin. Biofactors 2009; 35(1): 36-46.
Zeng WQ, Al-Yamani E, Acierno JS Jr et al. Biotin-responsive basal ganglia disease maps to 2q36.3 and is due to mutations in SCL19A3. Am J Hum Genet. 2005; 77(1): 16-26.

## Claims

1. Biotin for use thereof in the treatment of multiple sclerosis, wherein said treatment with biotin has a duration of at least 3 months, and wherein the multiple sclerosis is a primary or secondary progressive form of multiple sclerosis.

2. Biotin for use according to Claim 1, in which the daily amount of biotin administered to the patient is comprised between 50 and 700 mg.

3. Biotin for use according to any one of Claims 1 to 2, in which the daily amount of biotin administered to the patient is at least 100 mg.

4. Biotin for use according to any one of Claims 1 to 3, in which the daily amount of biotin administered to the patient is at least 150 mg.

5. Biotin for use according to any one of Claims 1 to 3, in which the daily amount of biotin administered to the patient is comprised between 100 mg and 300 mg.

6. Biotin for use according to any one of Claims 1 to 5, **characterized in that** it is a form suitable for oral administration.

7. Biotin for use according to Claim 6, **characterized in that** said form for oral administration, contains at least 40 mg of biotin.

8. Biotin for use according to Claim 7, **characterized in that** said form for oral administration, contains at least 100 mg of biotin.

9. Biotin for use according to any one of Claims 1 to 8, **characterized in that** it is in the form of gel capsules, tablets (optionally film-coated), lozenges or pills.

10. Biotin for use according to any one of Claims 1 to 9, **characterized in that** it is in the form of a composition containing biotin and excipients, without any other active ingredient.

11. Biotin for use according to Claim 10, **characterized in that** the excipients are chosen from the group consisting of talc, microcrystalline cellulose, lactose and mannose.

12. Biotin afor use ccording to any one of Claims 1 to 3, **characterized in that** it is a form suitable for injectable administration.

13. Biotin for use according to any one of claims 1 to 12, **characterized in that** it is in the form of a slow release composition.

## Patentansprüche

1. Biotin zur Verwendung bei der Behandlung von Multipler Sklerose, wobei die Behandlung mit Biotin eine Dauer von mindestens 3 Monaten aufweist und wobei die Multiple Sklerose eine primäre oder sekundäre progressive Form von Multipler Sklerose ist.

2. Biotin zur Verwendung nach Anspruch 1, bei dem die tägliche Menge an Biotin, die dem Patienten verabreicht wird, zwischen 50 und 700 mg liegt.

3. Biotin zur Verwendung nach einem der Ansprüche 1 bis 2, worin die tägliche Menge an Biotin, die dem Patienten verabreicht wird, mindestens 100 mg beträgt.

4. Biotin zur Verwendung nach einem der Ansprüche 1 bis 3, worin die tägliche Menge an Biotin, die dem Patienten verabreicht wird, mindestens 150 mg beträgt.

5. Biotin zur Verwendung nach einem der Ansprüche 1 bis 3, worin die tägliche Menge an Biotin, die dem Patienten verabreicht wird, zwischen 100 mg und 300 mg liegt.

6. Biotin zur Verwendung nach einem der Ansprüche 1 bis 5, **dadurch gekennzeichnet, dass** es eine zur oralen Verabreichung geeignete Form ist.

7. Biotin zur Verwendung nach Anspruch 6, **dadurch gekennzeichnet, dass** die Form zur oralen Verabreichung mindestens 40 mg Biotin enthält.

8. Biotin zur Verwendung nach Anspruch 7, **dadurch gekennzeichnet, dass** die Form zur oralen Verabreichung mindestens 100 mg Biotin enthält.

9. Biotin zur Verwendung nach einem der Ansprüche 1 bis 8, **dadurch gekennzeichnet, dass** es in Form von Gelkapseln, Tabletten (gegebenenfalls folienbeschichtet), Rauten oder Pillen vorliegt.

10. Biotin zur Verwendung nach einem der Ansprüche 1 bis 9, **dadurch gekennzeichnet, dass** es in Form einer Zusammensetzung vorliegt, die Biotin und Hilfsstoffe enthält, ohne einen anderen aktiven Bestandteil.

11. Biotin zur Verwendung nach Anspruch 10, **dadurch gekennzeichnet, dass** die Hilfsstoffe ausgewählt sind aus der Gruppe bestehend aus Talkum, mikrokristalliner Cellulose, Laktose und Mannose.

12. Verwendung von Biotin gemäß einem der Ansprüche 1 bis 3, **dadurch gekennzeichnet, dass** es eine Form ist, die für die injizierbare Verabreichung geeignet ist.

13. Biotin zur Verwendung nach einem der Ansprüche 1 bis 12, **dadurch gekennzeichnet, dass** es in Form einer Zusammensetzung mit langsamer Freisetzung vorliegt.

## Revendications

1. Biotine pour son utilisation dans le traitement de la sclérose en plaques, dans laquelle ledit traitement à la biotine a une durée d'au moins 3 mois, et dans laquelle la sclérose en plaques est une forme progressive primaire ou secondaire de sclérose en plaques.

2. Biotine pour son utilisation selon la revendication 1, dans laquelle la quantité quotidienne de biotine administrée au patient est comprise entre 50 et 700 mg.

3. Biotine pour son utilisation selon l'une quelconque des revendications 1 à 2, dans laquelle la quantité quotidienne de biotine administrée au patient est d'au moins 100 mg.

4. Biotine pour son utilisation selon l'une quelconque des revendications 1 à 3, dans laquelle la quantité quotidienne de biotine administrée au patient est d'au moins 150 mg.

5. Biotine pour son utilisation selon l'une quelconque des revendications 1 à 3, dans laquelle la quantité quotidienne de biotine administrée au patient est comprise entre 100 mg et 300 mg.

6. Biotine pour son utilisation selon l'une quelconque des revendications 1 à 5, **caractérisée en ce qu'**il s'agit d'une forme appropriée pour une administration orale.

7. Biotine pour son utilisation selon la revendication 6, **caractérisée en ce que** ladite forme pour administration orale contient au moins 40 mg de biotine.

8. Biotine pour son utilisation selon la revendication 7, **caractérisée en ce que** ladite forme pour administration orale contient au moins 100 mg de biotine.

9. Biotine pour son utilisation selon l'une quelconque des revendications 1 à 8, **caractérisée en ce qu'**elle se présente sous forme de capsules de gel, de comprimés (éventuellement pelliculés), de pastilles ou de comprimés.

10. Biotine pour son utilisation selon l'une quelconque des revendications 1 à 9, **caractérisée en ce qu'**elle se présente sous la forme d'une composition contenant de la biotine et des excipients, sans aucun autre principe actif.

11. Biotine pour son utilisation selon la revendication 10, **caractérisée en ce que** les excipients sont choisis dans le groupe consistant en talc, cellulose microcristalline, lactose et mannose.

12. Biotine pour son utilisation selon l'une quelconque des revendications 1 à 3, **caractérisée en ce qu'**il s'agit d'une forme appropriée pour une administration injectable.

13. Biotine pour son utilisation selon l'une quelconque des revendications 1 à 12, **caractérisée en ce qu'**elle se présente sous la forme d'une composition à libération lente.
